(19) Europäisches Patentamt
European Patent Office
Office européen des brevets

(11) **EP 2 931 369 B1**

(12) **EUROPÄISCHE PATENTSCHRIFT**

(45) Veröffentlichungstag und Bekanntmachung des Hinweises auf die Patenterteilung:
**03.05.2017 Patentblatt 2017/18**

(21) Anmeldenummer: **13783019.6**

(22) Anmeldetag: **18.10.2013**

(51) Int Cl.:
***A61N 5/10*** *(2006.01)*

(86) Internationale Anmeldenummer:
**PCT/EP2013/071858**

(87) Internationale Veröffentlichungsnummer:
**WO 2014/090456 (19.06.2014 Gazette 2014/25)**

(54) **BESTRAHLUNGSPLANUNG EINER PARTIKELBESTRAHLUNG UNTER BERÜCKSICHTIGUNG EINER BEWEGUNG EINES ZIELVOLUMENS**

PLANNING THE IRRADIATION OF A PARTICLE BEAM WHILE TAKING INTO CONSIDERATION A MOVEMENT OF A TARGET VOLUME

PLANIFICATION D'IRRADIATION POUR UN PROCESSUS D'IRRADIATION AUX PARTICULES EN TENANT COMPTE D'UN DÉPLACEMENT D'UN VOLUME-CIBLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.12.2012 DE 102012112348**

(43) Veröffentlichungstag der Anmeldung:
**21.10.2015 Patentblatt 2015/43**

(73) Patentinhaber: **GSI Helmholtzzentrum für Schwerionenforschung GmbH**
**64291 Darmstadt (DE)**

(72) Erfinder:
• **BERT, Christoph**
**91080 Uttenreuth (DE)**
• **GEMMEL, Alexander**
**91052 Erlangen (DE)**
• **MÜSSIG, Dirk**
**63853 Mömlingen (DE)**
• **LÜCHTENBORG, Robert**
**48155 Münster (DE)**

(74) Vertreter: **Blumbach Zinngrebe**
**Patentanwälte**
**Alexandrastrasse 5**
**65187 Wiesbaden (DE)**

(56) Entgegenhaltungen:
**DE-A1-102008 027 485   DE-A1-102008 036 478**
**DE-A1-102009 055 902**

## Beschreibung

Gebiet der Erfindung

Die Erfindung betrifft ein Verfahren und eine Vorrichtung zur Bestrahlungsplanung sowie eine Beschleunigereinrichtung mit einem Partikelstrahl.

Hintergrund der Erfindung

Die Tumortherapie mit schweren Ionen hat sich im Laufe der letzten Jahrzehnte zu einem etablierten Verfahren zur Behandlung von Gewebe, insbesondere von Tumorerkrankungen, entwickelt. Die dabei gewonnenen Erfahrungen werden jedoch auch in rein technischen Gebieten angewendet, wie beispielsweise im Rahmen von Forschungsarbeiten oder Produktentwicklungen, wobei unbelebte Materie eingesetzt wird.

Gemeinsames Merkmal aller bekannten Verfahren ist hierbei, dass ein von einer Beschleunigeranlage bereitgestellter gebündelter Partikelstrahl mittels eines Hochenergiestrahltransportsystems zu einem oder mehreren Bestrahlungs- oder Behandlungsräumen geleitet wird. In dem Bestrahlungsraum wird ein zu bestrahlendes Zielvolumen positioniert und mit dem Partikelstrahl bestrahlt.

Es ist bekannt, dass sich ein zu bestrahlendes Zielvolumen bewegen kann. Beispielsweise kann in dem Zielvolumen ein Lungentumor angeordnet sein, der bei einem Atemzug des Patienten bewegt wird. Beispielsweise zur Untersuchung des Einflusses der Bewegung auf den Behandlungserfolg der Partikeltherapie kann die Bewegung mittels als Phantomen bezeichneten, nicht lebenden Modellkörpern nachgebildet werden und ein solches Phantom mit dem Partikelstrahl bestrahlt werden.

Eine besondere Herausforderung im Rahmen der Partikeltherapie ist es, eine möglichst homogene Verteilung der im Gewebe deponierten Strahlungsdosis zu erreichen. Ein Grund, weshalb eine homogene Dosisverteilung im Zielvolumen von besonderem Interesse ist, ist die Tatsache, dass die Zellen des im Zielvolumen befindlichen Tumors erst ab einer Schwelldosis mit ausreichender Sicherheit sterben, zugleich aber eine übermäßige Belastung des umliegenden gesunden Gewebes verhindert werden soll. So ist es nach wie vor schwierig, bei Bestrahlungsverfahren, bei denen eine Vielzahl von Einzelbestrahlungsdosen sukzessive an verschiedenen Zielpunkten im Zielvolumen deponiert werden soll, also bei einem gescannten Partikelstrahl, diese gewünschte homogene Dosisverteilung im Zielvolumen zu erreichen, wenn sich das Zielvolumen während der Bestrahlung bewegt. Die Verbesserung der Homogenität der Dosisverteilung im Zielvolumen ist daher nach wie vor Stand der Forschung.

Beispielsweise ist es bei einem gescannten Partikelstrahl eine Möglichkeit, die zu applizierende Strahlungsdosis auf mehrere Durchgänge zu verteilen, was als "Rescanning" bezeichnet wird. Hierbei werden die Zielpunkte des Zielvolumens mehrfach angefahren, so dass die zu applizierende Gesamtdosis sukzessive durch mehrere, wiederholt applizierte Einzeldosen während der Rescan-Durchgänge aufgebaut wird. Das wiederholte Anfahren der Zielpunkte mit Einzeldosen ermöglicht, den Einfluss der Bewegung auf die Gesamtdosisverteilung im Zielvolumen durch ein statistisches Mitteln über die Einzeldosen zu verringern. Mit anderen Worten können möglicherweise fehlerhaft deponierte Dosen statistisch betrachtet gemittelt werden und Bewegungen des Zielvolumens während der Bestrahlungsdauer können zumindest teilweise kompensiert werden.

Allerdings ist dabei in Kauf zu nehmen, dass gerade der Randbereich des Zielvolumens nicht so scharf von dem das Zielvolumen umgebenden Material, beispielsweise gesundes Gewebe, getrennt werden kann. Um sicherzugehen, dass möglichst in dem ganzen Zielvolumen die gewünschte Solldosis appliziert wird, werden typischerweise Sicherheitssäume um das Zielvolumen eingeführt, die das eigentlich zu bestrahlende klinische Zielvolumen deutlich vergrößern. Hierdurch muss aber ggf. gesundes Gewebe bestrahlt werden, um eine sichere Dosisabdeckung im Zielvolumen zu gewährleisten.

Des Weiteren ist es bekannt, im Rahmen des alternativ zu dem Rescan-Verfahren angewendeten Gating-Verfahrens die Bewegung des Zielvolumens zu verfolgen.

Die DE 10 2009 055 902 A1 lehrt zur Bestimmung des Bewegungsmodells die Berücksichtigung eines 4D-Computertomogramms.

Allgemeine Beschreibung der Erfindung

Die Erfindung hat sich daher die Aufgabe gestellt, ein Verfahren bereit zu stellen, welches die Bestrahlungsplanung eines Zielvolumens vereinfacht und dabei die vorgenannten Nachteile des Standes der Technik mindert oder beseitigt.

Eine weitere Aufgabe der Erfindung ist es, das Bestrahlungsergebnis robuster gegenüber Veränderungen, insbesondere Bewegungen, des Zielvolumens zu machen.

Die Erfindung soll ferner die Zeitdauer der gesamten Behandlung bzw. Dosisapplikation verringern.

Schließlich ist es Aufgabe der Erfindung, um das Zielvolumen benachbartes Material, wie insbesondere gesundes Gewebe oder zu schützende Organe, besser auszusparen und weniger gesundes Gewebe zu bestrahlen.

Die Aufgabe der Erfindung wird durch den Gegenstand der unabhängigen Ansprüche gelöst. Vorteilhafte Weiterbildungen der Erfindung sind in den Unteransprüchen definiert.

Die Bestrahlungsplanung eines in einem Körper angeordneten Zielvolumens, die Schritt für Schritt den Bestrahlungsplan für die spätere Bestrahlung mit dem Partikelstrahl erstellt, wird mit der vorliegenden Erfindung anhand der folgenden Schritte definiert:

Zunächst wird ein Zielvolumen festgelegt, welches typischerweise in einem Körper angeordnet ist. Das Zielvolumen ist dabei in einem unbelebten Körper, wie beispielsweise einer Materialprobe, einem Phantom oder einem Versuchsaufbau angeordnet . Insbesondere befinden sich in dem Zielvolumen, Tumorzellen. Die vorliegend benutzten Fachbegriffe, beispielsweise Zielvolumen, Zielpunkt, Solldosis, Dosisverteilung etc., werden fachüblich verwendet und sind in dem als Leitfaden akzeptierten ICRU Report 50 (mit dem Addendum Report 62) definiert.

Das Zielvolumen wird in einem Referenzzustand der Bewegung des Körpers und/oder des Zielvolumens festgelegt. Mit anderen Worten handelt es sich um ein bewegtes Zielvolumen, wobei in der Bewegung des Zielvolumens eine Referenzphase erkennbar oder definierbar ist. Ein besonders einfaches Beispiel einer Bewegung mit Referenzphase ist die Lungenrespiration, also eine zyklische Atembewegung. Als Referenzphase der Atmung kann beispielsweise das Ende der Exspiration oder der Beginn der Inspiration festgelegt werden. Beispielsweise entspricht ein Respirationszyklus der Summe der (vorauszuplanenden) Bewegungsphasen der Bewegung des Zielvolumens. Es sind aber auch andere bewegte Zielvolumen eingeschlossen, bei denen zumindest eine Referenzphase der Bewegung definierbar und somit die Bestrahlung mit dem Partikelstrahl zu der Bewegung korrelierbar ist.

Das Zielvolumen wird in eine Vielzahl einzeln mit dem Partikelstrahl anfahrbarer Zielpunkte unterteilt. Um die Zielpunkte des Zielvolumens mit dem Partikelstrahl einzeln anzufahren, wird der Partikelstrahl vorzugsweise kurz vor dem Austreten aus dem Vakuum, also kurz vor dem Auftreffen auf das Zielvolumen, mit kleinen Richtungsänderungen abgelenkt. Bevorzugt können dafür Ablenkmagnete wie die der Rasterscaneinrichtung der Anmelderin verwendet werden.

Mit anderen Worten wird in das Zielvolumen ein Punktraster aus Zielpunkten mit definierten Punktabständen gelegt. Bevorzugt werden die Punktabstände in den Richtungen definiert, die der longitudinalen Hauptausbreitungsrichtung des Partikelstrahls bzw. den dazu transversalen Richtungen entsprechen. Die Hauptausbreitungsrichtung des Partikelstrahls ist dabei diejenige gedachte Achse, auf der der nichtabgelenkte Partikelstrahl verläuft. Das Punktraster kann demnach also bevorzugt einem kartesischen Koordinatensystem entsprechen, wobei die Richtungen x und y der horizontalen bzw. vertikalen Senkrechten mit Bezug auf die Hauptausbreitungsrichtung des Partikelstrahls entsprechen, und ferner die Richtung z der (longitudinalen) Eindringtiefe des Partikelstrahls in dem Material entspricht.

Bei jedem Einzelbestrahlungsvorgang, wobei ein Zielpunkt des Zielvolumens von dem Partikelstrahl der Partikel-Bestrahlungsanlage mit einer Einzeldosis belegt wird, wird der gesamte Eintrittskanal des Partikelstrahls in dem Körper, in dem sich das Zielvolumen befindet, mit einem als Vordosis bezeichneten Dosisbetrag belegt. In dem aufwändigen Bestrahlungsplanungsverfahren müssen all die entstehenden Vordosen berücksichtigt und zur Berechnung der Gesamtdosis herangezogen werden. So kann es beispielsweise vorteilhaft sein, zunächst das distale Ende eines Zielvolumens zu bestrahlen, wobei der proximalere Teil des Zielvolumens bereits mit einer Vordosis belastet wird. Anschließend kann das Zielvolumen beispielsweise aus der Gegenrichtung bestrahlt werden, so dass der von dieser Seite aus distale Teil des Zielvolumens eine weitere Teildosis erhält. Alternativ kann in weiteren Rescan-Durchgängen das Zielvolumen aus der gleichen Richtung weiter bestrahlt werden und der proximalere Teil mit einer schwächeren Dosis, also insbesondere mit einer geringeren Partikelzahl, bestrahlt werden, so dass die Solldosis je Zielpunkt schließlich erreicht wird und die Gesamtdosis im Zielvolumen möglichst homogen verteilt ist. Im Idealfall entspricht die Gesamtdosisverteilung über das Zielvolumen einer Stufenfunktion. Dies ist naturgemäß im Praxisfall nur schwer zu erreichen.

Den Zielpunkten wird jeweils eine Solldosis, d.h. der Plan- oder Sollwert der an dem jeweiligen Zielpunkt zu deponierenden Bestrahlungsdosis, zugeordnet. Mit anderen Worten berechnet die Bestrahlungsplanung die an den jeweiligen Zielpunkten zu deponierende Solldosis und schreibt das Ergebnis in den Bestrahlungsplan.

Die Solldosis wird ferner auf eine Anzahl von Rescan-Durchgängen aufgeteilt, in denen jeweils zumindest ein Teil der Zielpunkte angefahren wird. Bevorzugt werden in jedem Rescan-Durchgang des Partikelstrahls alle Zielpunkte angefahren, ggf. sind die Rescan-Durchgänge auch identisch zueinander bezüglich des Anfahrens der Zielpunkte. Besonders dann, wenn in jedem Rescan-Durchgang dieselben Zielpunkte angefahren werden oder bei identischen Rescan-Durchgängen tritt der statistische Mittelungseffekt des Rescanning-Verfahrens in vorteilhafter Weise auf.

Mit anderen Worten wird also bereits in der Bestrahlungsplanung eine Anzahl von Rescan-Durchgängen festgelegt, in denen die Zielpunkte des Zielvolumens jeweils angefahren werden. Ein wesentlicher Vorteil des Aufteilens der Solldosis auf Rescan-Durchgänge ist es, dass wenn es im späteren Verlauf bei der Applikation der Dosis an einem der Zielpunkte beispielsweise zu einer unerwarteten Bewegung des Zielvolumens kommt, wodurch die zu applizierende Dosis nicht an dem Zielpunkt sondern fehlerhaft deponiert wird, dies statistisch durch die vorgehenden oder folgenden Rescan-Durchgänge zumindest teilweise ausgleichbar ist.

In einem einfachen Fall bewegen sich die Zielpunkte des bewegten Zielvolumens richtungsgleich in dem Körper, so dass das Zielvolumen im Wesentlichen unverformt eine Translation vollzieht und das Punktgitter der Zielpunkte einheitlich bleibt. Die Bewegung der Zielpunkte des Zielvolumens kann aber auch relativ zueinander erfolgen. Beispielsweise kann das Zielvolumen gedehnt, gequetscht, gestreckt oder anderweitig umgeformt werden, und auch Dichteschwankungen im Zielvolumen oder in dem umliegenden Material können für die Eindringtiefe des Partikelstrahls von Bedeutung sein, so dass sich die Zielpunkte entsprechend verschieben. Auch eine Überlagerung einer Translation mit einer Verformung des Zielvolumens ist zu berücksichtigen und wird ebenfalls als Bewegung des Zielvolumens verstanden.

Dabei steht ein Bewegungsmodell zur Verfügung, welches Rückschlüsse auf die zu erwartende Bewegung des Zielvolumens und/oder der Bewegung der Zielpunkte des Zielvolumens zulässt. Als Grundlage zur Bestimmung des Bewegungsmodells wird ein Patientenmodell, eine mathematische (Bewegungs)Funktion, und/oder eine Atmungsstudie berücksichtigt. Die Atmungsstudie kann an einem Einzelzielvolumen oder an einer ganzen Reihe von Zielvolumen, beispielsweise in Form einer Patientenerhebung, bestimmt werden, so dass eine mittlere erwartete Bewegung der Zielpunkte des Zielvolumens, beispielsweise einem Lungentumor, vorausberechenbar ist. Auch die weiteren Grundlagen zur Bestimmung des Bewegungsmodells erlauben es, eine mittlere zu erwartende Bewegung der Zielpunkte des Zielvolumens vorauszuberechnen. Insbesondere wird zur Bestimmung des Bewegungsmodells das gewichtete Mittel der Dosisbeiträge aus den erwarteten Bewegungszuständen berücksichtigt.

Das Bewegungsmodell, das die Bewegung der Zielpunkte des Zielvolumens vorhersagt, kann dazu herangezogen werden, im Rahmen der Bestrahlungsplanung die Abweichung der bewegungsfreien Solldosis von derjenigen Dosisdeposition zu erfassen, die unter Berücksichtigung der Bewegung zu erwarten ist. Das bedeutet, dass eine Auswertung des Bewegungsmodells bereits im Vorfeld der Bestrahlung, also im Rahmen der Bestrahlungsplanung, die Homogenität der Dosisdeposition und/oder die Konformität, d.h. die Randschärfe der Dosisdeposition, im Falle eines Rescanning-Verfahrens mit einem gescannten Partikelstrahl erhöhen kann. In vorteilhafter Weise wird das Bewegungsmodell auch hinsichtlich der Erkennung von Bewegungsphasen herangezogen und die Bestrahlung mit dem Partikelstrahl mit zumindest einer Bewegungsphase, insbesondere der Referenzphase, zeitlich korreliert. So kann der Vorteil des Rescanning-Verfahrens, nämlich die Zeitersparnis gegenüber alternativen Verfahren, erhalten bleiben, indem die Zeitspanne der Bestrahlung zumindest mehrere Bewegungsphasen umfasst oder sogar den gesamten Bewegungsprozess des Zielvolumens umfasst. Die Homogenität der mittels Rescanning erzielbaren Dosisverteilung sowie die Schärfe der Dosisdeposition im Zielvolumenrandbereich, die Konformität, lässt sich darüber hinaus signifikant verbessern, so dass Sicherheitssäume um das Zielvolumen, die ggf. in gesundem Gewebe oder gar in Risikoorganen (OAR - Organs At Risk) angesiedelt werden müssen, verkleinert werden können oder gar ganz wegfallen können.

Durch eine Korrelation der Bestrahlung mit der Bewegung der Zielpunkte des Zielvolumens kann die zu erwartende mittlere Bewegung der Zielpunkte des Zielvolumens berücksichtigt werden, indem die Solldosis an jedem Zielpunkt auf Grundlage der gefundenen Abweichung korrigiert wird. Im Einzelnen lässt sich die Solldosis beispielsweise durch eine Änderung der an dem Zielpunkt zu deponierende Partikelzahl einstellen, wobei die Gesamtpartikelzahl auf die Rescan-Durchgänge aufgeteilt wird und wobei die einzelnen Partikelzahlen je Rescan-Durchgang auch unterschiedlich sein können. Beispielsweise kann es vorteilhaft sein, bei einer bekannten Bewegung des Zielvolumens, insbesondere bei Dichteschwankungen des Zielvolumens, in einer Phase der Bewegung eine hohe Partikelzahl an dem Zielpunkt zu deponieren, in einer weiteren Phase der Bewegung dagegen nur eine niedrige Partikelzahl, um so durch statistische Ausgleichwirkung die optimale Homogenität der Dosisverteilung im Zielvolumen zu erhalten. Mit anderen Worten kann das Anfahren von ausgewählten Zielpunkten des Zielvolumens mit dem Bewegungszustand des Zielvolumens korreliert werden. Durch die Berücksichtigung der Bewegung bei der Bestrahlungsplanung kann insgesamt der Randbereich der Dosisdeposition im Zielvolumen schärfer definiert werden und die Strahlungsbelastung des das Zielvolumen umgebenden Materials, wie insbesondere gesundes Gewebe oder zu schützende Organe, verringert werden.

Schließlich werden aus den gewonnenen Daten der Zielpunkte, der Bewegung und der resultierenden Abweichung der Zielpunkte des Zielvolumens auf Grund der Bewegung Steuerparameter für die Bestrahlungsanlage erzeugt, so dass die gewünschte korrigierte Solldosis je Zielpunkt mit der Bestrahlungsanlage deponiert werden kann. Insbesondere umfassen die Steuerparameter die pro Zielpunkt und Rescan-Durchgang in dem Zielvolumen zu deponierende Partikelzahl.

Die Zielpunkte können zu Isoenergieschichten des Partikelstrahls zugeordnet sein. Hierdurch können die Zielpunkte eine Isoenergieschicht mit konstanter Partikelenergie, also insbesondere unveränderten Beschleunigereinstellungen, bestrahlt werden. Mit anderen Worten ist es vorteilhaft, die zu Isoenergieschichten zugeordneten Teile der Zielpunkte jeweils konsekutiv anzufahren, um die Beschleunigereinstellungen seltener zu ändern und somit die Zeitdauer des Gesamtbestrahlungsvorgangs zu verkürzen.

Die erwarteten Dosisverteilungen für die Zielpunkte des Zielvolumens können insbesondere für die Isoenergieschichten des Partikelstrahls berechnet werden. Bevorzugt werden die Isoenergieschichten jeweils mit dem Auftreten eines festgelegten Bewegungszustands, also beispielsweise dem Referenzzustand der Bewegung, angefahren. Mit anderen Worten kann der Start (das Anfahren) einer Isoenergieschicht mit einer bestimmten Bewegungsphase synchronisiert werden. Dies kann die Robustheit der Dosisdeposition gegenüber Bewegungen des Zielvolumens weiter erhöhen.

Ferner liegt es im Rahmen der Erfindung, ein Verfahren zur Bestrahlung eines in einem Körper angeordneten bewegten Zielvolumens mit einer Partikel-Bestrahlungsanlage mittels Rescanning vorzustellen. Das Bestrahlungsverfahren nutzt insbesondere die im Vorfeld der Bestrahlung erzeugten Bestrahlungsplanungsdaten, indem der Bestrahlungsplan in eine Steuereinheit der Bestrahlungsanlage geladen wird.

Zur Detektion der Bewegung des Zielvolumens im Verlauf der Bestrahlung wird eine Bewegungserfassungseinrichtung herangezogen, so dass die vorausberechnete Bewegung im Verlauf der Bestrahlung auch anhand der tatsächlichen Bewegung verifiziert und/oder korrigiert werden kann. Anhand der Daten der Bewegungserfassungseinrichtung können demgemäß weitere Steuerparameter für die Bestrahlungsanlage generiert werden, mittels derer die auf Basis der Bestrahlungsplanung vorausgeplante Solldosis korrigiert werden kann.

Die anhand der Bestrahlungsparameter erzeugten Steuerparameter und die anhand der Bewegungserfassungseinrichtung erzeugten weiteren Steuerparameter werden schließlich dazu herangezogen, den Partikelstrahl zu steuern, so dass das Zielvolumen mit dem Partikelstrahl unter Berücksichtigung der Bewegung des Zielvolumens bestrahlt werden kann.

Im Rahmen der Erfindung wird auch eine Bestrahlungsanlage bereitgestellt, die geeignet ist, die Schritte der oben genannten Verfahren auszuführen.

Im Folgenden wird die Erfindung anhand von Ausführungsbeispielen und unter Bezugnahme auf die Figuren näher erläutert, wobei gleiche und ähnliche Elemente teilweise mit gleichen Bezugzeichen versehen sind und die Merkmale der verschiedenen Ausführungsbeispiele miteinander kombiniert werden können.

Kurzbeschreibung der Figuren

Es zeigen:

Fig. 1    eine Übersicht über den Aufbau einer Bestrahlungsanlage, die einen Partikelstrahl im Rahmen von Rescanning-Verfahren bereitstellt

Fig. 2    eine schematische Darstellung von zur Steuerung einer Bestrahlungsanlage verwendeten Komponenten

Fig. 3    eine schematische Darstellung einer Ablenk- und Modulationseinrichtung sowie eine Bestrahlung eines Zielvolumens

Fig. 4    Durch das erfindungsgemäße Verfahren erhaltene Dosisverteilungsänderung im Zielvolumen

Fig. 5    Überblick über Schritte der erfindungsgemäßen Verfahren

Detaillierte Beschreibung der Figuren

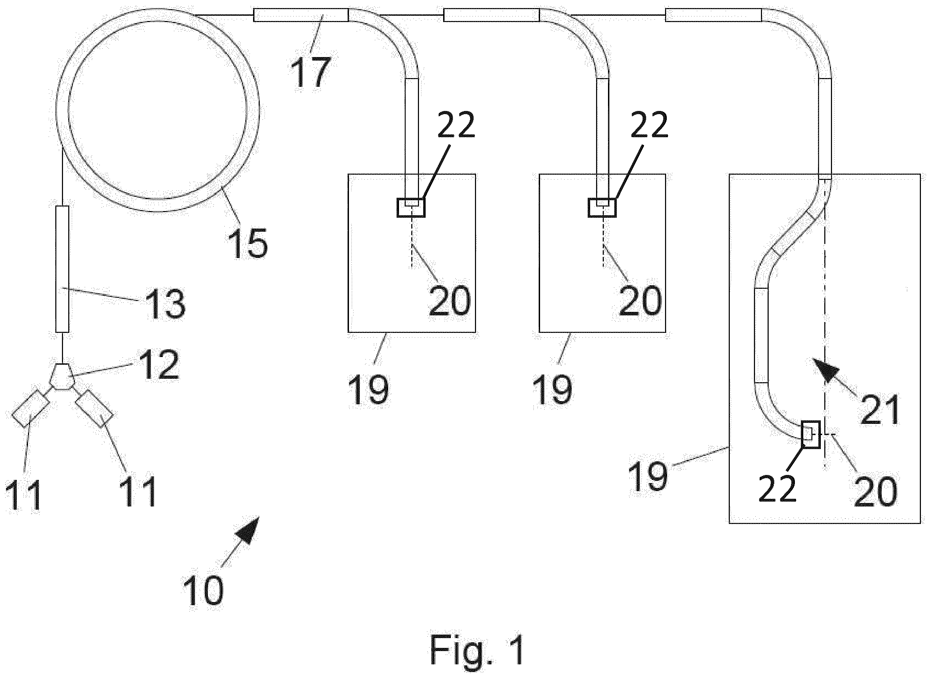

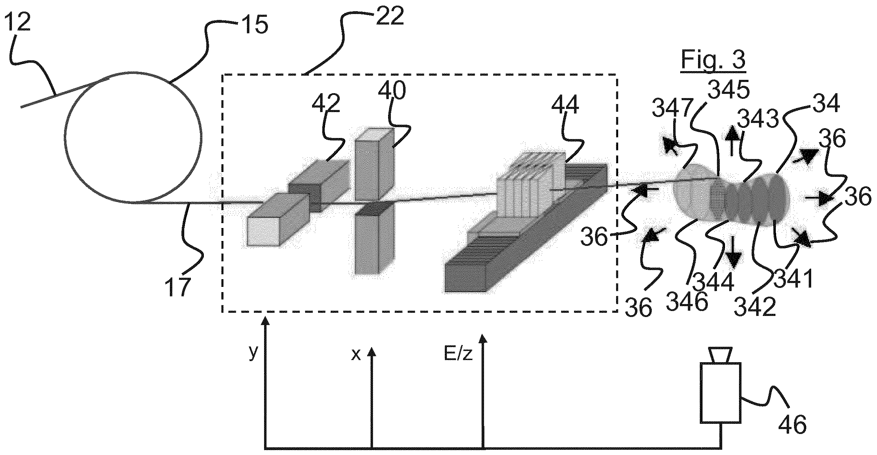

Figur 1 zeigt den schematischen Aufbau einer an sich bekannten Partikeltherapieanlage 10. Die Partikeltherapieanlage 10 erzeugt und beschleunigt geladene Partikel, die in Form eines Partikelstrahls 20 zur weiteren Verwendung bereitgestellt werden und mittels einer Strahlführung 17 in ein definierbares Zielvolumen 34 (siehe Figur 3) lenkbar sind. Das Zielvolumen 34 enthält beispielsweise im Rahmen einer Tumortherapie einen Tumor, es kann aber auch zu wissenschaftlichen Zwecken, Tierversuchen, Modell- und Materialproben und allgemein zur Erforschung des Partikelstrahls und/oder der Partikeltherapie ein Zielvolumen 34 definiert sein, welches unbelebtes Material und/oder Zellkulturen enthält. Die Partikeltherapieanlage 10 wird auch zur Bestrahlung von Phantomen mit dem Partikelstrahl 20 eingesetzt, mittels welchen eine Vielzahl an Bestrahlungsparametern vor und/oder nach einer erfolgten Bestrahlung bzw. Behandlung eines Patienten verifiziert werden kann.

Die Partikel werden in dem in Figur 1 gezeigten Beispiel in einer der zwei Ionenquellen 11 erzeugt und vorbeschleunigt. Die Ionenquellen 11 können insbesondere verschiedenste Teilchen von Protonen bis Uran bereitstellen. Aufgrund ihrer für die Partikeltherapie vorteilhaften Eigenschaften wie der charakteristischen (teilchenabhängigen) Wechselwirkung mit Materie und der Eindringtiefe werden bevorzugt Protonen, Pionen, Heliumionen oder besonders bevorzugt Kohlenstoffionen eingesetzt. Ganz allgemein werden bevorzugt Hadronen als Partikel eingesetzt. Mittels einer Niedrigenergiestrahlführung 12 werden die Partikel in einen Vorbeschleuniger 13, im gezeigten Fall der Linearbeschleuniger UNILAC 13 (Universal Linear Accelerator), eingefädelt. Der Linearbeschleuniger 13 beschleunigt die Partikel auf eine erste Energiestufe und bündelt die Partikel zu einem Partikelstrahl 20. Mit der ersten Energiestufe werden die Partikel schließlich in die Beschleunigereinheit 15, z.B. wie dargestellt ein Synchrotron, oder auch ein Zyklotron, mit einer weiteren Niedrigenergiestrahlführung 12 eingefädelt und dort weiter bis auf die für die jeweilige Anwendung einstellbare Extraktionsenergie beschleunigt. Die Strahlführung 17 führt den Partikelstrahl 20 schließlich an das gewünschte Ziel, genannt "Target", in einem Messraum 19 oder Therapieraum 21, wo der Partikelstrahl 20 mit einem typischen Strahldurchmesser von 3 bis 30 Millimetern angewendet werden kann bzw. dort bereitgestellt wird.

Zur genauen Positionierung des Partikelstrahls 20 zum Anfahren eines Zielpunkts 30 eines Zielvolumens 34 in einem Körper 77 befindet sich im Mess- oder Bestrahlungsraum 19 oder Therapieraum 21 eine Ablenk- und Modulationseinrichtung 22 zur transversalen, also horizontalen und vertikalen, Ablenken des Partikelstrahls 20 und zur Energiemodulation zum schnellen Variieren der Partikelstrahlenergie, die die Eindringtiefe des Partikelstrahls 20 bestimmt (longitudinale Variation). Da hiermit ein ganzes Raster an Zielpunkten in einem Zielvolumen sukzessive angefahren werden kann und das sukzessive Anfahren der Zielpunkte als "Scannen" bezeichnet wird, wird die Einrichtung als Rasterscaneinrichtung 22 bezeichnet. Die Erfindung ist aber nicht auf die Verwendung einer Rasterscaneinrichtung 22 beschränkt, vielmehr können die Bestrahlungsverfahren Spotscan, kontinuierliche Bestrahlung und Rasterscan eingesetzt werden.

Die Reihenfolge des Anfahrens der Zielpunkte 30 des Zielvolumens 34 wird in einem Bestrahlungsplan festgehalten, der ferner weitere wesentliche Parameter enthalten kann, wie insbesondere die Parameter des Zielvolumens 34 und/oder eine zu erwartende Bewegung des Zielvolumens 34. Einer der wesentlichen Vorzüge der Rasterscaneinrichtung 22 ist, dass diese die Möglichkeit bereitstellt, den Partikelstrahl 20 dauerhaft auf das Zielvolumen 34 zu richten, ohne diesen für das Anfahren des jeweiligen Zielpunkts 30 einzeln zu aktivieren.

Die gesamte Partikeltherapieanlage 10 wird schließlich von einem Beschleunigerkontrollsystem gesteuert, welches insbesondere die Beschleunigereinheit 15 sowie die Strahlführung 17 steuert und Messdaten zur Überwachung von Strahlparametern sammelt. Gegebenenfalls können die Parameter zur Steuerung der Partikeltherapieanlage 10 basierend auf dem Bestrahlungsplan eingestellt werden, so dass der Bestrahlungsplan auch die Einstelldaten zur Steuerung der Partikeltherapieanlage 10 umfasst.

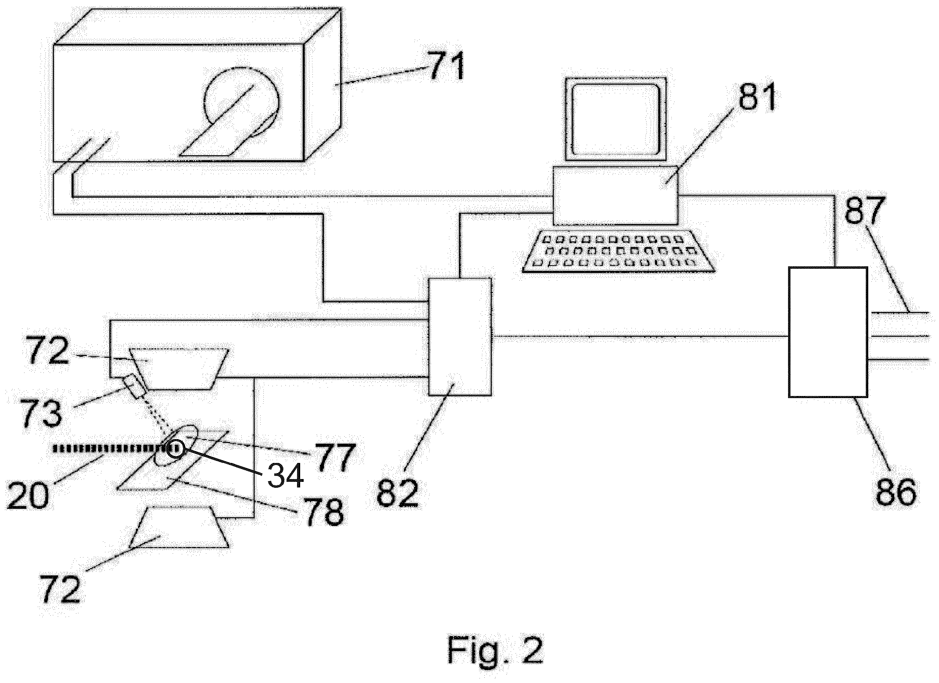

Figur 2 zeigt eine schematische Darstellung von an sich bekannten Einrichtungen, die bei der Erstellung des Bestrahlungsplanes, also der Bestrahlungsplanung, zum Erzeugen eines Datensatzes, der Zielpunkte 30 in einem Zielvolumen 34 in einem Körper 77 definiert, und bei der Steuerung einer Bestrahlungsanlage 10, wie sie beispielsweise anhand der Figur 1 dargestellt wurde, verwendet werden können.

Mittels eines Computer-Tomographen oder Kernspin-Tomographen 71 oder mittels anderer diagnostischer Einrichtungen können Lage und Ausdehnung eines zu bestrahlenden Tumors oder eines anderen Zielvolumens 34 ermittelt werden. Daten von dem Tomographen 71 werden unmittelbar oder nach einer Aufbereitung durch eine Vorrichtung 81 zum Erstellen eines Datensatzes verarbeitet. Die Vorrichtung 81 ist beispielsweise ein Arbeitsplatz-Computer, eine Workstation oder ein anderer Computer. Optional ist die Vorrichtung 81 ferner durch ihre Benutzerschnittstelle, Software oder andere Merkmale dafür geeignet, dass medizinisches Personal dort das Zielvolumen 34, die zu applizierenden Dosen, die Aufteilung derselben auf mehrere Fraktionen, die Richtung der Bestrahlung und andere Details der Partikeltherapie definiert.

Der zu bestrahlende Körper 77 kann mit verschieden ausgebildeten Überwachungseinrichtungen vor, während oder nach der Bestrahlung durch die Partikeltherapieanlage 10 überwacht werden. Beispielsweise sind eine PET-Kamera 72 (PET = Positronen-Emissions-Tomographie) und/oder ein Abstandssensor 73 zur Erfassung eines zu bestrahlenden Körpers 77, der auf einer Lagerfläche 78 gelagert ist, vorgesehen. Die PET-Kamera 72 und/oder der Abstandssensor 73 und die Lagerfläche 78 können innerhalb eines der oben anhand der Figur 1 dargestellten Bestrahlungsräume 19 angeordnet sein. In diesem Fall können mittels der PET-Kamera 72 und/oder des Abstandssensor 73 die durch einen Partikelstrahl 20 erzeugte Dosis sowie Bewegungen des bestrahlten Körpers 77 erfasst werden. Alternativ sind die PET-Kamera 72, der Abstandssensor 73 und die Lagerfläche 78 außerhalb eines Bestrahlungsraums angeordnet. Alternativ oder zusätzlich kann der Körper 77 mittels einer Fluoroskopie-Einrichtung, einer Röntgen-Einrichtung, eines Ultraschallsensors, eines Atemgürtels und/oder anderer externer Sensoren überwacht werden.

Daten vom Tomographen 71, von der PET-Kamera 72 und vom Abstandssensor 73 können von einer Einrichtung 82 zum Bestimmen von einem oder mehreren Bewegungsparametern verarbeitet werden. Mittels der Einrichtung 82 können vor einer Bestrahlung oder während einer Bestrahlung Bewegungen von Teilbereichen des Körpers 77 (beispielsweise aufgrund von Atmung oder Herzschlag) quantitativ erfasst werden. Der oder die von der Einrichtung 82 bestimmten Bewegungsparameter können von der Vorrichtung 81 zum Erstellen eines Datensatzes berücksichtigt werden.

Zur Berücksichtigung bei der Erstellung eines Datensatzes eignen sich insbesondere Daten über die Amplituden typischer und/oder periodischer Bewegungen oder über einen Zusammenhang zwischen der räumlichen Lage des Zielvolumens und/oder einer von außen, beispielsweise mittels des Abstandssensors 73 erfassbaren Größe. Alternativ oder zusätzlich können von der Einrichtung 82 bestimmte Parameter bzw. Daten direkt von einer Steuerung 86 zur Steuerung einer Bestrahlungsanlage 10, wie sie anhand der Figur 1 dargestellt wurde, verarbeitet werden. Dazu eignen sich besonders Daten, die während der Bestrahlung von der PET-Kamera 72 oder dem Abstandssensor 73 erfasst werden. In die Steuerung der Anlage 10 durch die Steuerungseinheit 86 geht ferner der von der Vorrichtung 81 erstellte Datensatz ein. Über Steuerleitungen 87 oder auf andere Weise ist die Steuerungseinheit 86 mit der Bestrahlungsanlage 10 gekoppelt.

Der anhand der Figur 1 dargestellte Grundaufbau einer Bestrahlungsanlage 10 ist typisch für viele Partikeltherapieanlagen und andere Bestrahlungsanlagen. Die nachfolgend beschriebenen Ausführungsbeispiele sind sowohl in Zusammenhang mit der anhand der Figur 1 dargestellten Bestrahlungsanlage und den anhand der Figur 2 dargestellten Einrichtungen als auch mit anderen Bestrahlungsanlagen und Einrichtungen einsetzbar.

Figur 3 zeigt schematisch eine Bestrahlung eines Zielvolumens. Die Beschleunigereinheit 15 stellt den Partikelstrahl 20 bereit, welcher mit zwei Magnetscanpaaren 40, 42 transversal zur Strahlrichtung des Partikelstrahls, also lateral über das Zielvolumen 34 gerastert wird. Die Zielpunkte 30 des Zielvolumens 34 definieren das Punktraster des Zielvolumens, wobei die Zielpunkte in einer Vielzahl an Isoenergieschichten, im gezeigten Beispiel die Isoenergieschichten 341 bis 347, angeordnet sind. Die Isoenergieschichten 341 bis 347 werden sukzessive mit dem Partikelstrahl 20 abgerastert. In der Ausführungsform der Figur 3 wird gerade die Isoenergieschicht 345 lateral abgerastert. Eine Bewegung des Zielvolumens 34 wird durch die Pfeile 36 symbolisiert. Diese kann mittels einer Bewegungserfassungseinrichtung 46, die die Bewegung des Körpers 77 erfasst, erfasst werden.

Der Partikelstrahl 20 deponiert beim Anfahren eines Zielpunktes 30 eine Dosisverteilung, wobei die Dosisverteilung typischerweise abhängig von Profil und Durchmesser des eingesetzten Partikelstrahls ist. So kann der Partikelstrahl einen runden Querschnitt aufweisen, so dass auch die Dosisverteilung konzentrisch vom Strahlmittelpunkt aus betrachtet nach außen hin abnimmt.

Für den Fachmann ist es nicht notwendig, eine dreidimensionale Dosisdeposition, die ggf. auch Zielpunkte 30 benachbarter Isoenergieschichten 344, 346 betrifft, die Berechnung der dreidimensionalen Vordosen und die Auswahl der Bestrahlungsreihenfolge der Isoenergieschichten im Detail zu klären, da dieser vielmehr bereits daraus und aus den Figuren auf den vollständigen Bestrahlungsvorgang rückschließen kann und insbesondere den Bestrahlungsplan für alle Isoenergieschichten 341 bis 347 gleichermaßen anpassen kann. So kann das Verfahren anhand der Beschreibung und der Figuren auf räumlich im Zielvolumen 34 verteilte Zielpunkte 30 übertragen werden.

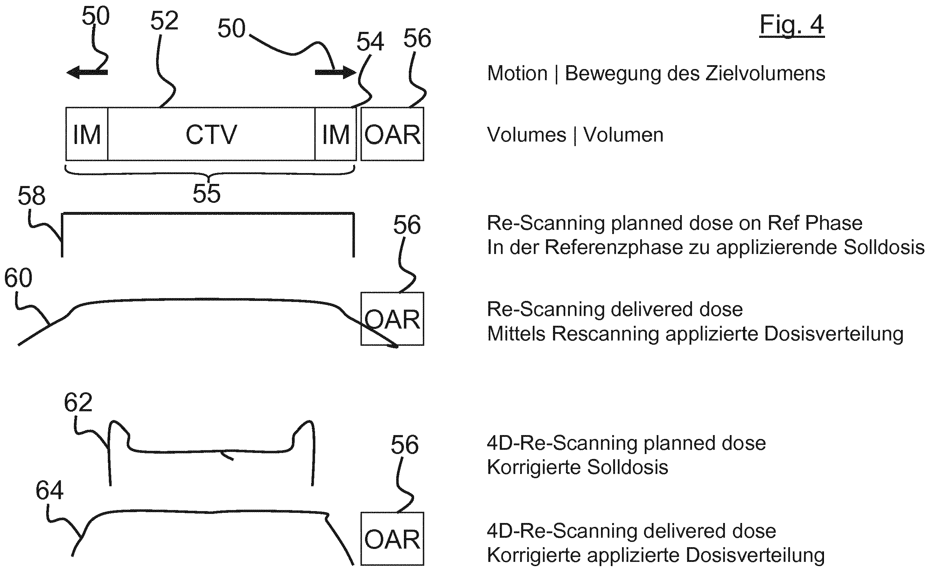

Figur 4 zeigt den Einfluss der Berücksichtigung der Bewegung auf die Dosisverteilung im Bereich des Zielvolumens. Die Pfeile 50 symbolisieren die Bewegung, die im gezeigten Fall eine periodische Atembewegung darstellt. Der innere Bereich zeigt das klinische Zielvolumen 52 (CTV Clinical Target Volume), der von Sicherheitssäumen 54 (IM Internal Margins) eingeschlossen ist. Klinisches Zielvolumen 52 und Sicherheitssäume 54 bilden gemeinsam das Zielvolumen 55. Das mit den Ausführungsformen der Figuren 2 und 3 beschriebene Zielvolumen 34 kann dem Zielvolumen 55 entsprechen. Ein Risikobereich 56 (OAR Organs At Risk), der besonders schonend zu berücksichtigen ist, der also von einer möglichst geringen Strahlungsdosis belastet werden soll, ist dem Zielvolumen 55 benachbart angeordnet. Die Stufenfunktion 58 zeigt die für gewöhnlich gewünschte Solldosisverteilung innerhalb des Zielvolumens 55. Mithin soll in dem ganzen Zielvolumen 55, das das klinische Zielvolumen 52 sowie die Sicherheitssäume 54 umfasst, eine homogene, möglichst niedrige aber letale Dosis deponiert werden. Die auf Grundlage einer solchen unkorrigierten Solldosisverteilung typischerweise im Gewebe deponierte Dosisverteilung zeigt die Verteilungskurve 60. Eine signifikante Dosisleistung wird typischerweise auch im Risikobereich 56 deponiert.

Die korrigierte Verteilungskurve 62 zeigt schließlich die korrigierte Solldosisverteilung 62 nach der Korrektur durch die zu erwartende mittlere Bewegung der Zielpunkte 30 des Zielvolumens 34, 55, wie es die Erfindung vorschlägt. Die Berücksichtigung der Bewegung kann daher die Dosisverteilung im Gewebe besser an das Ziel der Bestrahlung, nämlich der ausreichend hohen Dosisdeposition im klinischen Zielvolumen 52, anpassen. Die früher bei bewegten Zielvolumen eingeführten Sicherheitssäume 54 können daher unter Verwendung der erfindungsgemäßen Verfahren schmaler werden oder ggf. vollständig weggelassen werden. Eine bessere Schonung des Risikobereichs 56 findet darüber hinaus statt.

Die im Gewebe anhand der korrigierten Solldosisverteilung 62 deponierte Dosisleistung zeigt die Verteilungskurve 64. Wesentliches Ziel der Optimierung ist es, die Solldosisverteilung an ein bewegtes Zielvolumen 55 anzupassen und somit den Bereich, in dem eine Dosisleistung deponiert wird, möglichst auf das klinische Zielvolumen 52 zu beschränken. Dies lässt sich ganz anschaulich der Figur 4 entnehmen. Die erfindungsgemäß korrigierte Solldosisverteilung 62 erzeugt eine Verteilungskurve 64 der Dosisdeposition, die deutlich steilere Flanken aufweist und nicht so weit von dem klinischen Zielvolumen 52 entfernt liegen, somit also weniger umliegendes Material wie gesundes Gewebe oder Risikobereiche 56 betreffen.

In dem Beispiel der Figur 4 wird in dem Risikobereich 56 deutlich erkennbar eine geringere Dosis bzw. sogar keinerlei Strahlungsdosis deponiert. Auch in dem bisher definierten Sicherheitssaum 54 ist die deponierte Dosisleistung geringer, so dass die Sicherheitssäume schmaler ausgebildet oder im Idealfall weggelassen werden können. Mit dem erfindungsgemäßen Verfahren kann es gelingen, eine homogenere und/oder konformere korrigierte Dosisverteilung 64 zu erhalten auch unter dem Einfluss der Bewegung 36, 50 des Zielvolumens 34, 55, so dass das Bestrahlen des Zielvolumens ggf. auch außerhalb der Referenzphase der Bewegung durchgeführt werden kann, womit eine deutliche Zeitersparnis erhalten wird.

Im Folgenden wird ein Beispiel einer mathematischen Funktion diskutiert, welche Grundlage zur Bestimmung des Bewegungsmodells sein kann. Um die gesamte Dosis möglichst nahe an die "verordnete" Solldosis zu bringen, was Ziel der Optimierung mittels des Bewegungsmodells ist, betrachten wir im Folgenden die Bestimmung der Partikelzahl für jeden Rasterpunkt (ohne Berücksichtigung eines zu schonenden Risikobereichs 56). Beispielsweise ist für eine 3D Optimierung das Minimum der folgenden Funktion F(i,N$_j$) zu suchen:

$$F(i, N_j) = \sum_i (D_{\text{plan}} - D_{i,\text{ref}}(\sum_j N_j))^2$$

Wobei $D_{\text{plan}}$ die Solldosis darstellt und $D_{i,\text{ref}}(\sum_j N_j)$ die an einem Zielpunkt i, 30 in der Referenzphase der Bewegung deponierte Dosis in Abhängigkeit von der an allen Zielpunkten j, 30 deponierten Partikelzahlen N$_j$ bezeichnen. Auf Basis dieser Funktion lässt sich allerdings nur eine Dosisverteilung erhalten, wie sie der Verteilungskurve 60 entspricht. Mit anderen Worten entspricht diese Funktion der Optimierung eines statischen Tumors, welche für klassisches Rescanning verwendet wird und die Berücksichtigung von Sicherheitssäumen um den Tumor erfordert. Dies führt zu vermehrter Bestrahlung gesunden Gewebes.

Im neu vorgeschlagenen Verfahren zur Bestimmung der Bewegung wird die an einem Zielpunkt i, 30 deponierte Dosis aus dem gewichteten Mittel der Dosisbeiträge aus allen erwarteten Bewegungszuständen m berechnet. Die zu minimierende Funktion könnte beispielhaft folgendermaßen aussehen:

$$F(i, N_j, P_m) = \sum_i (D_{\text{plan}} - \sum_m P_m \cdot D_{i,m}(\sum_j N_j))^2$$

wobei $D_{i,m}(\sum_j N_j)$ die an dem Zielpunkt i, 30 in Bewegungszustand m deponierte Dosis und $P_m$ die Wahrscheinlichkeit angibt, das Zielvolumen 34, 55 im Bewegungszustand m anzutreffen. Mit anderen Worten wird die Bewegung des Zielvolumens mittels des Bewegungsmodells explizit berücksichtigt. Anhand dieser Funktion ist es möglich, die voraussichtliche Dosisdeposition jedes Rasterpunktes vorherzusagen, die für jede Phase der Bewegung berechenbar ist und über die Wahrscheinlichkeitsfunktion gewichtet werden kann. Dies ermöglicht demnach die Optimierung anhand einer tatsächlich zu erwartenden 4D-Dosisdeposition, statt der bisherigen stationären 3D-Dosisdeposition. Es handelt sich demnach um eine echte 4D-Optimierung, die besonders die Konformität der Dosis erhöhen kann.
Im Allgemeinen kann diese Wahrscheinlichkeitsfunktion nicht nur vom Bewegungszustand m, sondern auch von der Position des Zielpunkts j, 30 abhängen. Dies wäre z.B. der Fall, wenn Veränderungen der Bewegungstrajektorie schon vor der Bestrahlung bekannt wären, oder künstlich induziert werden. Zum Beispiel kann der Bewegungszustand mit Beginn des Anfahrens jeder Isoenergieschicht festgelegt werden oder es kann die Teilchenintensität während der Bestrahlung variiert werden. In diesem Fall könnte die Funktion folgendermaßen aussehen:

$$F(i, N_j, P_{m,j}) = \sum_. (D_{\text{plan}} - \sum \sum P_{m,j} \cdot D_{i,m}(N_j))^2$$

Die Applizierung der geplanten Solldosis erfolgt also über die Optimierung, die, anders als im konventionellen Rescanning, Informationen aus allen Bewegungszuständen explizit berücksichtigt, statt allein über die Festlegung der Sicherheitssäume 54 gewährleistet zu werden. Der Bereich, in dem Dosis deponiert wird, kann so verringert werden. Dies wird insbesondere dazu führen, dass zu schonende Risikoorgane 56, die in der Nähe des klinischen Zielvolumens 52 lokalisiert sind, besser geschont werden können. Die Entscheidung, ob die Sicherheitssäume 54 reduziert werden oder ob auf sie ggf. sogar verzichtet werden kann, kann entweder manuell getroffen werden oder entsprechend in einer automatischen Optimierung angepasst werden. Auch ist eine Verwendung von konventionellen Sicherheitssäumen 54 mit dem erfindungsgemäßen Verfahren denkbar, um beispielsweise bei Atemmustern, die stark von einer Gleichverteilung der Bewegungszustände abweichen, eine verbesserte Dosisdeposition zu erreichen, oder die in Risikoorganen 56 deponierte Dosis inklusive Bewegungseinfluss besser abschätzen zu können.

Aus den Wahrscheinlichkeitsfunktionen der möglichen zeitlichen Entwicklung der Bewegungszustände lässt sich eine mittlere Bewegung der Zielpunkte des Zielvolumens berechnen. Die mittlere berechnete Bewegung der Zielpunkte des Zielvolumens kann schließlich bei der Bestrahlungsplanung berücksichtigt werden, um die Konformität der Dosisdeposition zu erhöhen.

Auch kann das Verfahren ggf. mit dem Gating-Verfahren kombiniert werden, so dass die Bestrahlung nur in einer ausgesuchten Untermenge aller Bewegungszustände stattfindet. Hierdurch könnte z.B. im Vergleich zum reinen Gating mit Zielvolumen 55 ein größeres Gating-Fenster toleriert und somit die Gesamtbestrahlungsdauer verringert werden. Die Wahl des Gating-Fensters kann manuell erfolgen oder Teil der Optimierung sein.

Vorteilhaft kann dieses Verfahren benutzt werden, indem zusätzlich während der Bestrahlung der Bewegungsverlauf gemessen wird. Wird eine zu große Abweichung in der Verteilung der Bewegungsphasen von der angenommenen Verteilung festgestellt, können verschiedene Gegenmaßnahmen ergriffen werden, beispielsweise: Pausieren (Gating) der Bestrahlung während der dominanten Bewegungszustände, Neuplanung für die aktuelle oder zukünftige Fraktion oder Felder, Modulation der eingestrahlten Teilchenintensität (Erhöhung während subdominanter bzw. Verringerung während dominanter Bewegungszustände) sowie kontrollierte Atmung des Patienten, z.B. über Atem-Coaching oder künstliche Beatmung.

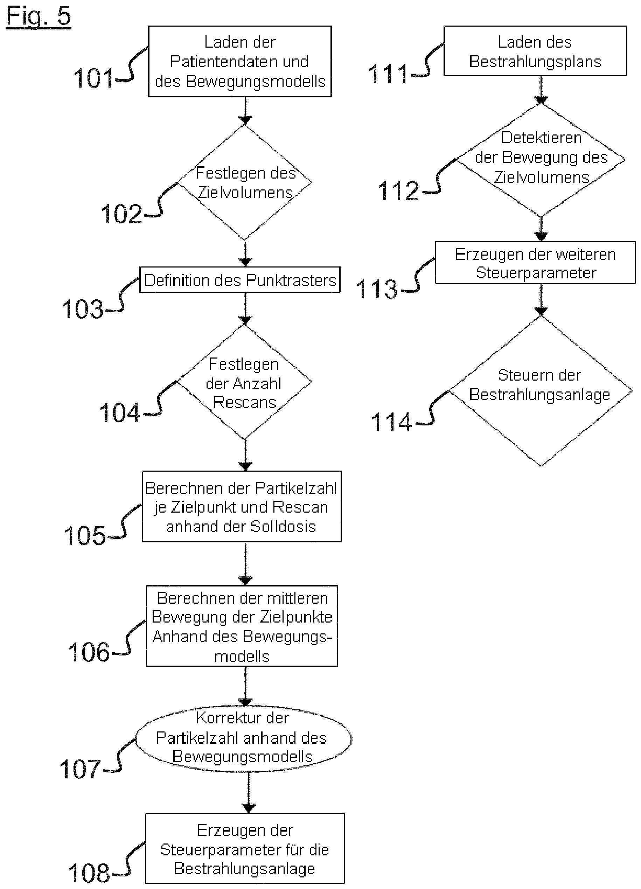

Figur 5 zeigt linkerhand acht Schritte des der Bestrahlung vorausgehenden Bestrahlungsplanungsverfahrens. Zunächst werden mit Schritt 101 Patientendaten sowie das anzuwendende Bewegungsmodell geladen. Die Patientendaten enthalten insbesondere Informationen über die zu applizierende Dosisleistung, die Ausdehnung des Zielvolumens 34, 55, Informationen über Risikobereiche 56 sowie Informationen über Dichteverläufe im Körper 77, so dass die Partikelenergien und die einzelnen Dosisverteilungen berechnet werden können. Anhand der Patientendaten wird mit Schritt 102 das Zielvolumen 34, 55 definiert und mit Schritt 103 ein Punktraster, also die Zielpunkte 30 des Zielvolumens, definiert.

Mit Schritt 104 wird gemäß dem Beispiel der Figur 5 die Anzahl Rescan-Durchgänge festgelegt. Die Solldosis 58 wird je Zielpunkt 30 auf die Rescan-Durchgänge aufgeteilt.

In Schritt 105 wird die Partikelzahl je Zielpunkt 30 berechnet, die mit dem Partikelstrahl 20 der Beschleunigeranlage 10 zu deponieren ist. Die Partikelzahl je Zielpunkt 30 kann anhand der zu deponierenden Solldosis 58 berechnet werden.

Mit Schritt 106 wird das gewählte Bewegungsmodell auf die Solldosisverteilung 58 im Zielvolumen 34, 55 angewendet und die mittlere Bewegung der Zielpunkte 30 anhand des Bewegungsmodells berechnet. Eine Korrektur der Partikelzahl findet mit Schritt 107 statt, wobei anhand des Bewegungsmodells eine korrigierte Solldosis 62 berechnet wird.

Schließlich werden mit Schritt 108 die auf Grundlage des Bewegungsmodells korrigierten Steuerparameter für die Bestrahlungsanlage erzeugt und sind somit im Bestrahlungsplan für eine spätere Bestrahlung abrufbar.

Das Verfahren zur Bestrahlung eines Zielvolumens 34, 55 kann die folgenden, ebenfalls in Figur 5 skizzierten Schritte umfassen. Zunächst wird mit Schritt 111 der Bestrahlungsplan in eine Steuereinheit 86 zur Steuerung der Bestrahlungsanlage 10 geladen, der bereits die Patientendaten, die Daten des Zielvolumens und des Punktrasters sowie die korrigierte Solldosisverteilung 62 vorbereitet enthält.

Mittels einer Bewegungserfassungseinrichtung 82, die die Bewegungen des Zielvolumens 34, 55 quantitativ erfassen kann, wird mit Schritt 112 die Bewegung des Zielvolumens 34, 55 bevorzugt während des gesamten Bestrahlungsprozesses detektiert. Mit der Bewegungserfassungseinrichtung 82 kann im Übrigen ggf. auch eine Kontrolle der korrekten Bestrahlung durchgeführt werden.

Mit Schritt 113 werden auf Grundlage der mit der Bewegungserfassungseinrichtung 82 erzeugten tatsächlichen Bewegungsdaten des Zielvolumens 34, 55 weitere Steuerparameter erzeugt, anhand der die Steuerung der Bestrahlungsanlage 10 kontinuierlich korrigiert werden kann, falls die tatsächlichen Bewegungsdaten von den anhand des Bewegungsmodells vorausberechneten Bewegungsdaten abweichen. Eine ggf. vorzunehmende Korrektur der Steuerung der Bestrahlungsanlage wird mit Schritt 114 durchgeführt, mit welchem die Bestrahlungsanlage schließlich unter Berücksichtigung der Steuerparameter und der weiteren Steuerparameter gesteuert wird.

Es ist dem Fachmann ersichtlich, dass die vorstehend beschriebenen Ausführungsformen beispielhaft zu verstehen sind, und die Erfindung nicht auf diese beschränkt ist, sondern in vielfältiger Weise variiert werden kann, ohne die Erfindung zu verlassen. Ferner ist ersichtlich, dass die Merkmale unabhängig davon, ob sie in der Beschreibung, den Ansprüchen, den Figuren oder anderweitig offenbart sind auch einzeln wesentliche Bestandteile der Erfindung definieren, selbst wenn sie zusammen mit anderen Merkmalen gemeinsam beschrieben sind.

Bezugszeichenliste

| | |
|---|---|
| 10 | Bestrahlungsanlage |
| 11 | Ionenquelle |
| 12 | Niedrigenergiestrahlführung |
| 13 | Vorbeschleuniger |
| 15 | Beschleunigereinheit |
| 17 | Strahlführung |
| 19 | Messraum |
| 20 | Partikelstrahl |
| 21 | Therapieraum |

| 22 | Ablenk- und Modulationseinrichtung |
|----|-----|
| 30 | Zielpunkt |
| 34 | Zielvolumen |
| 36 | Pfeil |
| 40, 42 | Scannermagnete zur lateralen Ablenkung des Partikelstrahls |
| 44 | Doppelkeilsystem zur longitudinalen Ablenkung (=Abbremsung) des Partikelstrahls |
| 46 | Bewegungserfassungseinrichtung |
| 50 | Pfeil zur Verdeutlichung der Bewegung |
| 52 | Klinisches Zielvolumen |
| 54 | Sicherheitssaum (IM) |
| 56 | Risikobereich oder Risikoorgan (OAR) |
| 58 | Verteilungskurve einer einfachen geplanten Solldosisverteilung |
| 60 | Verteilungskurve einer herkömmlichen Dosisverteilung |
| 62 | Verteilungskurve einer bewegungskorrigierten Solldosisverteilung |
| 64 | Verteilungskurve einer bewegungskorrigierten Dosisverteilung |
| 71 | Computer- oder Kernspintomograph |
| 72 | PET-Kamera |
| 73 | Abstandssensor |
| 77 | Körper |
| 78 | Lagerfläche |
| 81 | Vorrichtung zum Erstellen eines Datensatzes |
| 82 | Einrichtung zur quantitativen Erfassung von Bewegungen |
| 86 | Steuerung |
| 87 | Steuerleitung |
| 101 bis 108 | Schritte des Planungsverfahrens |
| 111 bis 114 | Schritte des Bestrahlungsverfahrens |

**Patentansprüche**

1. Verfahren zur Bestrahlungsplanung für die Bestrahlung eines in einem Körper (77) angeordneten bewegten Zielvolumens (34, 55) mit einer Partikel-Bestrahlungsanlage (10) mittels Rescanning mit den Schritten:

   Festlegen (102) des Zielvolumens in einem Referenzzustand der Bewegung,
   Aufteilen (103) des Zielvolumens auf eine Mehrzahl einzeln mit einem Partikelstrahl anfahrbarer Zielpunkte (30),
   Festlegen (104) einer Anzahl von Rescan-Durchgängen, in denen die Zielpunkte des Zielvolumens jeweils angefahren werden,
   Berechnen (105) einer an den Zielpunkten des Zielvolumens jeweils zu deponierenden Solldosis (58),
   Berechnen (106) einer zu erwartenden mittleren Bewegung (36, 50) der Zielpunkte des Zielvolumens anhand eines Bewegungsmodells,
   Berücksichtigen der zu erwartenden mittleren Bewegung der Zielpunkte des Zielvolumens bei der Bestrahlungsplanung dergestalt, dass die Abweichung der erwarteten Dosisdeposition (60) von der Solldosis je Zielpunkt erfasst und die Solldosis je Zielpunkt auf Grundlage der Abweichung korrigiert wird und eine korrigierte Solldosis (62) erzeugt wird (107),
   Erzeugen (108) von Steuerparametern für die Bestrahlungsanlage, wobei die Steuerparameter eine zu applizierende Partikelzahl pro Zielpunkt und Rescan-Durchgang umfassen,
   wobei zur Bestimmung des Bewegungsmodells ein Patientenmodell, eine mathematische Funktion und/oder eine Atmungsstudie berücksichtigt wird und
   wobei die korrigierte Solldosis (62) in unterschiedlichen Bewegungszuständen auf die Rescan-Durchgänge aufgeteilt wird.

2. Verfahren zur Bestrahlungsplanung nach dem vorstehenden Anspruch,

   wobei das Bewegungsmodell des Zielvolumens (34, 55) unterschiedliche Bewegungszustände aufweist und die Solldosis je Zielpunkt gewichtet nach dem Bewegungszustand auf die Rescan-Durchgänge aufgeteilt wird.

3. Verfahren zur Bestrahlungsplanung nach einem der vorstehenden Ansprüche,

wobei das Bewegungsmodell des Zielvolumens (34, 55) zyklisch ist und der Referenzzustand wiederholt auftritt, so dass durch eine zeitliche Kopplung der Rescan-Durchgänge an das Bewegungsmodell die Solldosis je Zielpunkt gleichmäßig auf die Rescan-Durchgänge aufgeteilt wird.

4. Verfahren zur Bestrahlungsplanung nach einem der vorstehenden Ansprüche,

wobei die erwarteten Dosisverteilungen für die Zielpunkte (30) des Zielvolumens (34, 55) für Isoenergieschichten (341, 342, 343, 344, 345, 346, 347) des Partikelstrahls (20) berechnet werden.

5. Verfahren zur Bestrahlung eines in einem unbelebten Körper angeordneten bewegten Zielvolumens (34, 55) mit einer Partikel-Bestrahlungsanlage (10) mittels Rescanning mit den Schritten:

Erzeugen von Steuerparametern insbesondere mit einem Verfahren gemäß einem der vorstehenden Ansprüche, wobei die zu erwartende mittlere Bewegung der Zielpunkte (30) des Zielvolumens (34, 55) bei der Bestrahlungsplanung anhand eines Bewegungsmodells berücksichtigt wird, dergestalt, dass die Abweichung der erwarteten Dosisdeposition (60) von der Solldosis je Zielpunkt erfasst, die Solldosis je Zielpunkt auf Grundlage der Abweichung korrigiert und eine korrigierte Solldosis (62) erzeugt wird,
Laden (111) des im Vorfeld der Bestrahlung erzeugten Bestrahlungsplans mit den Steuerparametern in eine Steuereinheit (86),
Detektieren (112) der Bewegung des Zielvolumens im Verlauf der Bestrahlung mit einer Bewegungserfassungseinrichtung (82) und Erzeugen (113) von weiteren Steuerparametern mittels der Bewegungserfassungseinrichtung,
Steuern (114) des Partikelstrahls (20) mittels der im Bestrahlungsplan hinterlegten Steuerparameter und mittels der weiteren Steuerparameter zur Bestrahlung des Zielvolumens mit dem Partikelstrahl unter Berücksichtigung der Bewegung des Zielvolurnens,

wobei zur Bestimmung des Bewegungsmodells ein Patientenmodell, eine mathematische Funktion und/oder eine Atmungsstudie berücksichtigt wird und

wobei die korrigierte Solldosis (62) in unterschiedlichen Bewegungszuständen auf die Rescan-Durchgänge aufgeteilt wird.

6. Verfahren zur Bestrahlung nach vorstehendem Anspruch,

wobei die Partikelzahl des Partikelstrahls (20) mittels der im Bestrahlungsplan hinterlegten Steuerparameter und mittels der mit der Bewegungserfassungseinrichtung (82) erzeugten weiteren Steuerparameter variiert wird.

7. Verfahren zur Bestrahlung nach einem der Ansprüche 5 oder 6,

wobei die Bewegung des Zielvolumens (34, 55) unterschiedliche Bewegungszustände aufweist und die Solldosis je Zielpunkt gewichtet nach dem Bewegungszustand auf die Rescan-Durchgänge aufgeteilt wird.

8. Verfahren zur Bestrahlung nach einem der Ansprüche 5 bis 7,

wobei die Bewegung des Zielvolumens (34, 55) zyklisch ist und der Referenzzustand wiederholt auftritt, so dass durch eine zeitliche Kopplung der Rescan-Durchgänge an die Bewegung die Solldosis je Zielpunkt gleichmäßig auf die Rescan-Durchgänge aufgeteilt wird.

9. Verfahren zur Bestrahlung nach einem der Ansprüche 5 bis 8,

wobei die erwarteten Dosisverteilungen für die Zielpunkte (30) des Zielvolumens (34, 55) für Isoenergieschichten (341, 342, 343, 344, 345, 346, 347) des Partikelstrahls (20) berechnet werden.

10. Bestrahlungsanlage, umfassend:

eine Beschleunigereinheit (15),
eine Vorrichtung (81) zur Erstellung eines Datensatzes, die eingerichtet ist, berechnete Steuerparameter für eine Steuereinheit (86) bereitzustellen, mittels welchen unter Berücksichtigung eines Bewegungsmodells eine auf eine Anzahl Rescan-Durchgänge verteilte korrigierte Dosisverteilung (62) in einem Zielvolumen (34, 55) deponierbar ist,

eine Bewegungserfassungseinrichtung (82) zur Erfassung der Bewegung des Zielvolumens (34, 55) vor oder während der Bestrahlung mit dem Partikelstrahl, so dass mittels der Bewegungserfassungseinrichtung korrigierte weitere Steuerparameter erzeugbar sind und zur Steuerung der Bestrahlungsanlage bereitstellbar sind, eine Steuereinheit (86) zum Steuern der Beschleunigereinheit unter Heranziehen der Steuerparameter und der weiteren Steuerparameter zum Applizieren einer korrigierten Dosisverteilung (64) in dem Zielvolumen unter Berücksichtigung der Bewegung des Zielvolumens,

wobei zur Bestimmung des Bewegungsmodells ein Patientenmodell, eine mathematische Funktion und/oder eine Atmungsstudie berücksichtigt wird und

wobei die Steuereinheit (86) der Bestrahlungsanlage (10) ausgebildet ist, die korrigierte Solldosis (62) in unterschiedlichen Bewegungszuständen auf die Rescan-Durchgänge aufzuteilen.

**11.** Bestrahlungsanlage nach vorstehendem Anspruch,

wobei die Partikelzahl des Partikelstrahls (20) mittels der im Bestrahlungsplan hinterlegten Steuerparameter und mittels der mit der Bewegungserfassungseinrichtung (82) erzeugten weiteren Steuerparameter variiert wird.

**12.** Bestrahlungsanlage nach einem der Ansprüche 10 oder 11,

wobei die Steuereinheit (86) der Bestrahlungsanlage (10) ausgebildet ist, die Solldosis je Zielpunkt (30) gewichtet nach dem Bewegungszustand auf die Rescan-Durchgänge aufzuteilen.

**13.** Bestrahlungsanlage nach einem der Ansprüche 10 bis 12,

wobei die Bewegungserfassungseinrichtung (82) eine zyklische Bewegung des Zielvolumens (34, 55) anhand des Referenzzustands erfasst, und

wobei die Steuereinheit (86) der Bestrahlungsanlage (10) ausgebildet ist, durch eine zeitliche Kopplung der Rescan-Durchgänge an die Bewegung die korrigierte Solldosis (62) je Zielpunkt (30) gleichmäßig auf die Rescan-Durchgänge aufzuteilen.

**14.** Bestrahlungsanlage nach einem der Ansprüche 10 bis 13,

wobei die Steuereinheit (86) der Bestrahlungsanlage (10) ausgebildet ist, die Zielpunkte (30) des Zielvolumens (34, 55) in Isoenergieschichten (341, 342, 343, 344, 345, 346, 347) des Partikelstrahls (20) mit einer Partikelzahl entsprechend der korrigierten Dosisverteilung (62) anzufahren.

**15.** Bestrahlungsanlage nach dem Anspruch 14,

wobei die Steuereinheit (86) der Bestrahlungsanlage (10) ausgebildet ist, anhand der weiteren Steuerparameter der Bewegungserfassungseinrichtung (82) die Isoenergieschichten (341, 342, 343, 344, 345, 346, 347) jeweils mit dem Auftreten eines festgelegten Bewegungszustands mit dem Partikelstrahl (20) anzufahren.

**Claims**

**1.** A method for irradiation planning for the irradiation of a moving target volume (34, 55) located in a body (77), with a particle irradiation system (10) using rescanning, comprising the steps of:

defining (102) the target volume in a reference motion state;
dividing (103) the target volume into a plurality of target points (30) that can be individually targeted with a particle beam;
defining (104) a number of rescanning passes in which each of the target points of the target volume are targeted;
calculating (105) a nominal dose (58) to be deposited in each of the target points of the target volume;
calculating (106) a mean motion (36, 50) of the target points of the target volume to be expected based on a motion model;
taking into account the mean motion of the target points of the target volume to be expected in the irradiation planning in such a manner that the deviation of the expected dose deposition (60) from the nominal dose for each target point is determined and the nominal dose for each target point is corrected on the basis of the deviation, and a corrected nominal dose (62) is generated (107);
generating (108) control parameters for the irradiation system, wherein the control parameters include a particle count to be applied per target point and rescanning pass;
wherein a patient model, a mathematical function and/or a respiratory study is taken into account for determining the motion model; and
wherein the corrected nominal dose (62) in different states of motion is distributed to the rescanning passes.

2.  The method for irradiation planning according to the preceding claim,

wherein the motion model of the target volume (34, 55) includes different motion states and the nominal dose per target point is distributed to the rescanning passes, weighted according to the motion state.

3.  The method for irradiation planning according to any one of the preceding claims,

wherein the motion model of the target volume (34, 55) is cyclic and the reference state occurs repeatedly so that the nominal dose per target point is distributed evenly to the rescanning passes through a time-based coupling of the rescanning passes to the motion model.

4.  The method for irradiation planning according to any one of the preceding claims,

wherein the expected dose distributions for the target points (30) of the target volume (34, 55) are calculated for iso-energy layers (341, 342, 343, 344, 345, 346, 347) of the particle beam (20).

5.  A method for irradiating a moving target volume (34, 55) located in an inanimate body with a particle irradiation system (10) using rescanning, comprising the steps of:

generating control parameters, in particular with a method according to any one of the preceding claims, wherein the mean motion of the target points (30) of the target volume (34, 55) to be expected is taken into account during the irradiation planning by means of a motion model such that the deviation of the expected dose deposition (60) from the nominal dose per target point is determined, the nominal dose per target point is corrected based on the deviation and a corrected nominal dose (62) is generated;
loading (111) the irradiation plan with the control parameters generated prior to the irradiation into a control unit (86);
detecting (112) the motion of the target volume in the course of the irradiation using motion detection means (82) and generating (113) further control parameters using the motion detection means;
controlling (114) the particle beam (20) by means of the control parameters stored in the irradiation plan and by means of the further control parameters for irradiating the target volume with the particle beam while taking into account the motion of the target volume;
wherein for determining the motion model, a patient model, a mathematical function and/or a respiratory study is taken into account; and
wherein the corrected nominal dose (62) in different motion states is distributed to the rescanning passes.

6.  The method for irradiating according to the preceding claim,

wherein the particle count of the particle beam (20) is varied using the control parameters stored in the irradiation plan and using the further control parameters generated by the motion detection means (82).

7.  The method for irradiating according to any one of claims 5 or 6,

wherein the motion of the target volume (34, 55) includes different motion states and the nominal dose per target point is distributed to the rescanning passes, weighted according to the motion state.

8.  The method for irradiating according to any one of claims 5 to 7,

wherein the motion of the target volume (34, 55) is cyclic and the reference state occurs repeatedly so that the nominal dose per target point is distributed evenly to the rescanning passes through a time-based coupling of the rescanning passes to the motion model.

9. The method for irradiating according to any one of claims 5 to 8,

   wherein the expected dose distributions for the target points (30) of the target volume (34, 55) are calculated for iso-energy layers (341, 342,343, 344, 345, 346, 347) of the particle beam (20).

10. An irradiation system, comprising:

   an accelerator unit (15),
   a device (81) for generating a data set, adapted to provide calculated control parameters for a control unit (86) by means of which a corrected dose distribution (62) which is distributed to a number of rescanning passes can be deposited in a target volume (34, 55) while taking into account a motion model;
   motion detection means (82) for sensing the motion of the target volume (34, 55) prior to or during the irradiation with the particle beam so that corrected further control parameters can be generated by the motion detection means and can be provided for controlling the irradiation system;
   a control unit (86) for controlling the accelerator unit using the control parameters and the further control parameters to apply a corrected dose distribution (64) in the target volume while taking into account the motion of the target volume;
   wherein a patient model, a mathematical function and/or a respiratory study are taken into account for determining the motion model; and
   wherein the control unit (86) of the irradiation system (10) is adapted to distribute the corrected nominal dose in different motion states to the rescanning passes.

11. The irradiation system according to the preceding claim,

   wherein the particle count of the particle beam (20) is varied using the control parameters stored in the irradiation plan and using the further control parameters generated by the motion detection means (82).

12. The irradiation system according to any one of claims 10 or 11,

   wherein the control unit (86) of the irradiation system (10) is adapted to distribute the nominal dose per target point (30) to the rescanning passes, weighted according to the motion state.

13. The irradiation system according to any one of claims 10 to 12,

   wherein the motion detection means (82) detect a cyclic motion of the target volume (34, 55) on the basis of the reference state; and
   wherein the control unit (86) of the irradiation system (10) is adapted to evenly distribute the corrected nominal dose (62) per target point (30) to the rescanning passes through a time-based coupling of the rescanning passes to the motion.

14. The irradiation system according to any one of claims 10 to 13,

   wherein the control unit (86) of the irradiation system (10) is adapted to target the target points (30) of the target volume (34, 55) in iso-energy layers (341, 342, 343, 344, 345, 346, 347) of the particle beam (20) with a particle count corresponding to the corrected dose distribution (62).

15. The irradiation system according to claim 14,

   wherein the control unit (86) of the irradiation system (10) is adapted to target the iso-energy layers (341, 342, 343, 344, 345, 346, 347) with the particle beam (20) on the basis of the further control parameters of the motion detection means (82) upon the occurrence of a respective defined motion state.

**Revendications**

1. Procédé permettant d'établir des plans d'irradiation en vue de l'irradiation d'un volume cible (34, 55) disposé dans un corps (77), avec une installation d'irradiation par particules (10), par balayages multiples, comprenant les étapes suivantes :

définition (102) du volume cible dans un état de référence du mouvement,

répartition (103) du volume cible sur une pluralité de points cibles (30) pouvant être visés individuellement avec un faisceau de particules,

définition (104) d'un nombre de passages de balayages multiples, lors desquels les points cibles du volume cible sont respectivement visés,

calcul (105) d'une dose de consigne (58) à déposer respectivement aux points cibles du volume cible,

calcul (106) d'un mouvement moyen attendu (36, 50) des points cibles du volume cible, à l'aide d'un modèle de mouvement,

prise en compte du mouvement moyen attendu des points cibles du volume cible lors de la planification d'irradiation, de manière telle que l'écart du dépôt de dose attendu (60) par rapport à la dose de consigne soit détecté pour chaque point cible et la dose de consigne par point cible soit corrigée sur la base de l'écart et une dose de consigne corrigée (62) soit produite (107),

génération (108) de paramètres de commande pour l'installation d'irradiation, les paramètres de commande comportant un nombre de particules à appliquer par point cible et passage de balayage multiple,

selon lequel, pour la détermination du modèle de mouvement, on prend en compte un modèle de patient, une fonction mathématique et/ou une étude de la respiration, et

selon lequel la dose de consigne corrigée (62) est répartie sur les passages de balayage multiple, dans des états de mouvement différents.

2.  Procédé permettant d'établir des plans d'irradiation selon la revendication précédente,
    selon lequel le modèle de mouvement du volume cible (34, 55) présente différents états de mouvement, et la dose de consigne par point cible est répartie sur les passages de balayages multiples, en étant pondérée en fonction de l'état de mouvement.

3.  Procédé permettant d'établir des plans d'irradiation selon l'une des revendications précédentes,
    selon lequel le modèle de mouvement du volume cible (34, 55) est cyclique et l'état de référence apparaît de façon répétée, de sorte qu'un couplage temporel des passages de balayages multiples au modèle de mouvement a pour effet de répartir la dose de consigne par point cible de façon uniforme sur les passages de balayages multiples.

4.  Procédé permettant d'établir des plans d'irradiation selon l'une des revendications précédentes,
    selon lequel les distributions de doses attendues pour les points cibles (30) du volume cible (34, 55) sont calculées pour des couches isoénergétiques (341, 342, 343, 344, 345, 346, 347) du faisceau de particules (20).

5.  Procédé d'irradiation d'un volume cible (34, 55) en mouvement, disposé dans un corps inanimé, avec une installation d'irradiation par particules (10), par balayages multiples, comprenant les étapes suivantes :

génération de paramètres de commande, notamment à l'aide d'un procédé selon l'une des revendications précédentes, où le mouvement moyen attendu des points cibles (30) du volume cible (34, 55) est pris en compte à l'aide d'un modèle de mouvement lors de la planification d'irradiation, de manière telle que l'écart du dépôt de dose attendu (60) par rapport à la dose de consigne par point cible soit détecté, que la dose de consigne par point cible soit corrigée sur la base de l'écart et une dose de consigne corrigée (62) soit produite,

chargement (111) du plan d'irradiation, généré préalablement à l'irradiation, avec les paramètres de commande, dans une unité de commande (86),

détection (112) du mouvement du volume cible au cours de l'irradiation, à l'aide d'un dispositif de détection de mouvement (82), et génération (113) de paramètres de commande supplémentaires au moyen du dispositif de détection de mouvement,

commande (114) du faisceau de particules (20) au moyen des paramètres de commande enregistrés dans le plan d'irradiation et au moyen des paramètres de commande supplémentaires, en vue de l'irradiation du volume cible avec le faisceau de particules, en tenant compte du mouvement du volume cible,

selon lequel, pour la détermination du modèle de mouvement, on prend en compte un modèle de patient, une fonction mathématique et/ou une étude de la respiration, et

selon lequel la dose de consigne corrigée (62) est répartie sur les passages de balayages multiples, dans des états de mouvement différents.

6.  Procédé d'irradiation selon la revendication précédente,

selon lequel le nombre de particules du faisceau de particules est modifié au moyen des paramètres de commande enregistrés dans le plan d'irradiation et au moyen des paramètres de commande supplémentaires générés par le dispositif de détection de mouvement (82).

**7.** Procédé d'irradiation selon l'une des revendications 5 ou 6,
selon lequel le mouvement du volume cible (34, 55) présente différents états de mouvement et la dose de consigne par point cible est répartie sur les passages de balayages multiples, en étant pondérée en fonction de l'état de mouvement.

**8.** Procédé d'irradiation selon l'une des revendications 5 à 7,
selon lequel le mouvement du volume cible (34, 55) est cyclique et l'état de référence apparaît de façon répétée, de sorte qu'un couplage temporel des passages de balayages multiples au modèle de mouvement a pour effet de répartir la dose de consigne par point cible de façon uniforme sur les passages de balayages multiples.

**9.** Procédé d'irradiation selon l'une des revendications 5 à 8,
selon lequel les distributions de doses attendues pour les points cibles du volume cible (34, 55) sont calculées pour des couches isoénergétiques (341, 342, 343, 344, 345, 346, 347) du faisceau de particules (20).

**10.** Installation d'irradiation, comprenant :

une unité d'accélérateur (15),
un dispositif (81) pour établir un ensemble de données, qui est conçu pour mettre à la disposition d'une unité de commande (86) des paramètres de commande calculés qui permettent de déposer une distribution de dose corrigée (62), répartie sur un nombre de passages de balayages multiples, dans un volume cible (34, 55), en tenant compte d'un modèle de mouvement,
un dispositif de détection de mouvement (82) pour détecter le mouvement du volume cible (34, 55) avant ou pendant l'irradiation avec le faisceau de particules, de sorte que des paramètres de commande supplémentaires corrigés peuvent être générés au moyen du dispositif de détection de mouvement et être mis à disposition pour la commande de l'installation d'irradiation,
une unité de commande (86) pour commander l'unité d'accélérateur, en utilisant les paramètres de commande et les paramètres de commande supplémentaires, en vue de l'application d'une distribution de dose corrigée (64) dans le volume cible, en tenant compte du mouvement du volume cible,
sachant que pour la détermination du modèle de mouvement, on prend en compte un modèle de patient, une fonction mathématique et/ou une étude de la respiration, et
sachant que l'unité de commande (86) de l'installation d'irradiation (10) est conçue pour répartir la dose de consigne corrigée (62) sur les passages de balayages multiples, dans des états de mouvement différents.

**11.** Installation d'irradiation selon la revendication précédentes,
dans laquelle le nombre de particules du faisceau de particules est modifié au moyen des paramètres de commande enregistrés dans le plan d'irradiation et au moyen des paramètres de commande supplémentaires générés par le dispositif de détection de mouvement (82).

**12.** Installation d'irradiation selon l'une des revendications 10 ou 11,
dans laquelle l'unité de commande (86) de l'installation d'irradiation est conçue pour répartir la dose de consigne par point cible (30) sur les passages de balayages multiples, en la pondérant en fonction de l'état de mouvement.

**13.** Installation d'irradiation selon l'une des revendications 10 à 12,
dans laquelle le dispositif de détection de mouvement (82) détecte un mouvement cyclique du volume cible (34, 55) à l'aide de l'état de référence, et
dans laquelle l'unité de commande (86) de l'installation d'irradiation (10) est conçue pour répartir la dose de consigne corrigée (62) par point cible (30) de façon uniforme sur les passages de balayages multiples, à l'aide d'un couplage temporel des passages de balayages multiples au mouvement.

**14.** Installation d'irradiation selon l'une des revendications 10 à 13,
dans laquelle l'unité de commande (86) de l'installation d'irradiation (10) est conçue pour viser les points cibles (30) du volume cible (34, 55) dans des couches isoénergétiques (341, 342, 343, 344, 345, 346, 347) du faisceau de particules (20) avec un nombre de particules correspondant à la distribution de dose corrigée (62).

**15.** Installation d'irradiation selon la revendication 14,
dans laquelle l'unité de commande (86) de l'installation d'irradiation (10) est conçue pour viser les couches isoénergétiques (341, 342, 343, 344, 345, 346, 347) avec le faisceau de particules (20), à l'aide des paramètres de commande supplémentaires du dispositif de détection de mouvement (82), respectivement lors de l'apparition d'un état de mouvement défini.

Fig. 1

EP 2 931 369 B1

Fig. 2

Fig. 3

Fig. 4

50  52  50  54  56

Motion | Bewegung des Zielvolumens

| IM | CTV | IM | OAR |

Volumes | Volumen

58  55

56

Re-Scanning planned dose on Ref Phase
In der Referenzphase zu applizierende Solldosis

60

56

OAR

Re-Scanning delivered dose
Mittels Rescanning applizierte Dosisverteilung

62

56

4D-Re-Scanning planned dose
Korrigierte Solldosis

64

OAR

4D-Re-Scanning delivered dose
Korrigierte applizierte Dosisverteilung

EP 2 931 369 B1

Fig. 5

101 — Laden der Patientendaten und des Bewegungsmodells

111 — Laden des Bestrahlungsplans

102 — Festlegen des Zielvolumens

112 — Detektieren der Bewegung des Zielvolumens

103 — Definition des Punktrasters

113 — Erzeugen der weiteren Steuerparameter

104 — Festlegen der Anzahl Rescans

114 — Steuern der Bestrahlungsanlage

105 — Berechnen der Partikelzahl je Zielpunkt und Rescan anhand der Solldosis

106 — Berechnen der mittleren Bewegung der Zielpunkte Anhand des Bewegungsmodells

107 — Korrektur der Partikelzahl anhand des Bewegungsmodells

108 — Erzeugen der Steuerparameter für die Bestrahlungsanlage

**EP 2 931 369 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102009055902 A1